# EUROPÄISCHE PATENTSCHRIFT

(11) **EP 1 742 607 B1**
(45) Veröffentlichungstag und Bekanntmachung des Hinweises auf die Patenterteilung: **01.07.2009**
(21) Anmeldenummer: 05716399.0
(22) Anmeldetag: 26.03.2005
(51) Int. Cl.: A61K 8/06, A61K 8/37, A61K 8/81, A61Q 19/00

(54) **O/W-GEL-ZUSAMMENSETZUNGEN MIT ESTERN DES PENTAERYTHRITS ODER DESSEN OLIGOMEREN**
O/W-GEL-COMPOSITIONS HAVING PENTAERYTHRIOL ESTERS OR OLIGOMERS THEREOF
COMPOSITIONS HUILE/EAU AVEC DES ESTERS DE PENTAERYTHRITOL OU SES OLIGOMERES

(30) Priorität: 05.04.2004 DE 102004017221
(43) Veröffentlichungstag der Anmeldung: 17.01.2007
(73) Patentinhaber: Cognis IP Management GmbH, 40589 Düsseldorf (DE)
(72) Erfinder: GONDEK, Helga, 40589 Düsseldorf (DE); ISSBERNER, Ulrich, Ambler, PA 19002 (US); MITCHELL, Catherine, 40223 Düsseldorf (DE)
(86) Internationale Anmeldenummer: PCT/EP2005/003232
(87) Internationale Veröffentlichungsnummer: WO 2005/097055

(56) Entgegenhaltungen:
- EP-A- 0 179 416
- EP-A- 1 216 685
- WO-A-99/62468
- US-A- 3 976 789

## Beschreibung

### Gebiet der Erfindung

Die Erfindung betrifft spezifische O/W-Gelformulierungen, deren Verwendung als Hautpflegemittel und ein Verfahren zu deren Herstellung.

### Stand der Technik

Gel-Formulierungen auf Polyacrylatbasis sind dem Fachmann seit langem bekannt. Viele dieser Gelformulierungen betreffen tensidhaltige Mittel, die zur Körperreinigung eingesetzt werden. So beschreiben die WO 96/17591 und die WO 96/17592 tensidhaltige Formulierungen zur Hautreinigung, die durch polymere Gelbildner stabilisiert sind. Obgleich Gele einen sensorisch sehr leichten und angenehm kühlenden Eindruck vermitteln, werden sie üblicherweise nicht für die Formulierung von Hautpflegemittel eingesetzt, da sie zu geringe Pflegeeffekte aufweisen. Insbesondere Gel-Formulierungen auf Polyacrylatbasis zeigen keine nachhaltigen Pflegeeffekte. Gel-Formulierungen sind außerdem salzempfindlich, so dass sie beim Auftragen auf die Haut durch die Präsenz von Salzen häufig brechen.

Aufgabe der vorliegenden Erfindung war es, Gel-Formulierungen zur Verfügung zu stellen, die ein angenehmes, sensorisch leichtes Profil und gute Pflegeeffekte aufweisen und bei Applikation auf der Haut nicht brechen. Ein weiterer Aspekt der Aufgabe war es, möglichst irritationsfreie Formulierungen bereitzustellen.

Überraschenderweise wurde nun gefunden, dass sich diese Eigenschaften erreichen lassen, wenn man O/W-Gele formuliert, die eine Kombination von bestimmten Gelbildnern, Wachsen und Ölen enthalten.

### Beschreibung der Erfindung

Gegenstand der Erfindung sind daher O/W-Gelzusammensetzung enthaltend
(a) 0,05 - 5 Gew.-% wenigstens eines polymeren Gelbildners ausgewählt aus der Gruppe der Homopolymere oder Copolymere von Acrylsäure und/oder Acrylamid und deren Derivaten,
(b) 0,1 -10 Gew.-% wenigstens einer Wachskomponente mit einem Schmelzpunkt von wenigstens 30°C ausgewählt aus der Gruppe der Pentaerythritester, der Dipentaerythritester und/oder der Tripentaerythritester
(c) 1 - 30 Gew.-% wenigstens einer bei 25 °C flüssigen Ölkomponente und
(d) 60 - 95 Gew.-% Wasser.

Zusammensetzungen dieser Art sind gegenüber dem Salzgehalt auf der Haut wesentlich stabiler und hinterlassen bei der Anwendung ein glattes und weiches Gefühl mit sehr guten Pflegeeigenschaften. Sie sind leicht verteilbar, ziehen gut auf die Haut auf, hinterlassen ein relativ geringes öliges oder fettendes, sondern vielmehr samtiges Hautgefühl.

In einer bevorzugten Ausführungsform enthalten die erfindungsgemäßen Zusammensetzungen keine zusätzlichen anionischen und kationischen Tenside/Emulgatoren. Dadurch haben die Zusammensetzungen eine geringes Irritationspotential.

Die erfindungsgemäßen O/W-Gelzusammensetzungen weisen vorzugsweise bei 20°C eine Viskosität von 50000 bis 500000 mPa·s aufweist, gemessen mit einem Brookfield-Viskosimeter RVF, Spindel TE mit Helipath bei 4 Umdrehungen pro Minute.

### Gelbildner

Die Gelbildner sind ausgewählt aus der Gruppe der Homopolymere oder Copolymere von Acrylsäure und/oder Acrylamid und deren Derivaten oder aus einem beliebigen Gemisch dieser Substanzen. Häufig enthalten die marktüblichen Polymere auch ein nichtionisches Tensid/Emulgator. Zu den erfindungsgemäß einsetzbaren Gelbildnern gehören im Handel erhältliche Substanzen, wie beispielsweise Sepigel® 305, INCI: Polyacrylamid (und) C13-14 Isoparaffin (und) Laureth-7; Sepigel® 501, INCI: Acrylamid Copolymer (und) Mineralöl (und) C13-14 Isoparaffin (und) Polysorbat 85; Sepigel® 502, INCI: C13-14 Isoparaffin (und) Isostearyl Isostearat (und) Natrium Polyacrylat (und) Polyacrylamid (und) Polysorbat 60; Simulgel® 600, INCI: Acrylamid/Natrium Acryloyldimethyltaurat Copolymer (und) Isohexadecan (und) Polysorbat 80; Simulgel® 800, INCI: Natrium Polyacryloyldimethyl Taurat (und) Isohexadecan (und) Sorbitan Oleat; Simulgel® EG, INCI: Natrium Acrylat/Acryloyldimethyl Taurat Copolymer (und) Isohexadecan (und) Polysorbat 80; Simulgel® EG-SL, INCI: Natrium Acrylat/Acryloyldimethyl Taurat Copolymer (und) Polyisobuten (und) Caprylyl/Capryl Glucosid; Simulgel® NS, INCI: Hydroxyethyl Acrylat (und) Natrium Acryloyldimethyl Taurat Copolymer (und) Squalan (und) Polysorbat 60; Aristoflex® AVC, INCI: Ammonium Acryloyldimethyltaurat / VP Copolymer; Aristoflex® AVC-1, INCI: Ammonium Acryloyldimethyltaurat / Vinyl Formamid Copolymer; Aristoflex® HMB, INCI: Ammonium Acryloyldimethyltaurat / Beheneth-25 Methacrylat Copolymer; Salcare® SC91, INCI: Natrium Acrylate Copolymer (und) Mineralöl (und) PPG-1 Trideceth-6; Salcare® AST, INCI: Natrium Acrylate Copolymer (und) Glycin Soja (und) PPG-1 Trideceth-6; Pemulen® TR-1, INCI: Acrylat / C10-30 Alkyl Acrylat Crosspolymer; Pemulen® TR-2: Acrylat / C10-30 Alkyl Acrylat Crosspolymer; Carbopol® 980, INCI: Carbomer (z.B. Homopolymere von Acrylsäure vernetzt mit einem Allylether von Pentaerythritol, einem Allylether von Sucrose oder einem Allylether von Propylen); Carbopol® ETD 2020, INCI: Acrylat / C10-30 Alkyl Acrylate Crosspolymer; Carbopol® Ultrez 10, INCI: Carbomer; Rheocare® ATH, INCI: Natrium Polyacrylat (und) Ethylhexyl Stearat (und) Trideceth-6; Rheocare® ATC, INCI: Acrylamid / Natrium Acrylat Copolymer (und) Mineralöl (und) Trideceth-6;

Die Polymere können vernetzt oder unvernetzt sein. Vorzugsweise werden vernetzte Polymere eingesetzt. Erfindungsgemäß bevorzugt sind Polyacrylate und Polyacrylamide. Besonders bevorzugt sind Polyacrylate, insbesondere deren Natriumsalze. Ein erfindungsgemäß ganz besonders bevorzugtes Polymer ist unter dem Namen Cosmedia® SP und Cosmedia® SPL im Handel. Die Polymere werden erfindungsgemäß in Mengen von 0,05 - 5 Gew.-% bezogen auf die Gesamtzusammensetzung eingesetzt. Bevorzugt sind Mengen von 0,1 - 4 Gew.-%, insbesondere von 0,5 - 3 Gew.-% und ganz besonders bevorzugt von 0,5 - 2 Gew.-% bezogen auf die Gesamtzusammensetzung.

### Wachskomponenten

Unter dem Begriff Wachs werden üblicherweise alle natürlichen oder künstlich gewonnenen Stoffe und Stoffgemische mit folgenden Eigenschaften verstanden: sie sind von fester bis brüchig harter Konsistenz, grob bis feinkristallin, durchscheinend bis trüb und schmelzen oberhalb von 30°C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß einsetzbar ist eine Wachskomponente oder ein Gemisch von Wachskomponenten, die bei 30 °C oder darüber schmelzen und ausgewählt sind aus der Gruppe der Ester des Pentaerythrits, des Dipentaerythrits oder des Tripentaerythrits. Sie sind in den erfindungsgemäßen Zusammensetzungen in einer Gesamtmenge von 0,1 - 10 Gew.-% enthalten. In einer bevorzugten Ausführungsform der Erfindung liegt der Gehalt der Wachskomponente bei 0,2 - 5 Gew.-% bezogen auf die Gesamtzusammensetzung. Besonders bevorzugt sind Mengen von 0,5 - 4 Gew.-% und insbesondere 0,5 - 2 Gew.-% bezogen auf die Gesamtzusammensetzung. Ganz besonders bevorzugt ist ein Gehalt von 0,5 bis 1,5 Gew,.-% der Wachskomponente(n) bezogen auf die Gesamtzusammensetzung, da das sensorische Gesamtprofil in diesem Bereich optimal ist. Eine weitere bevorzugte Ausführungsform der O/W-Gelzusammensetzung ist dadurch gekennzeichnet, daß die Wachskomponente oder das Gemisch aus Wachskompoenten (b) einen Schmelzpunkt zwischen 40°C und 80 °C aufweist, vorzugsweise zwischen 40 und 60°C, da sich in diesem Bereich die besten sensorischen Effekte ergeben.

In einer weiteren bevorzugten Ausführungsform der Erfindung ist die Wachskomponente (b) ausgewählt aus der Gruppe der Ester gesättigter oder ungesättigter und/oder verzweigter oder unverzweigter C6-C24-Fettsäuren, vorzugsweise C14-C22-Fettsäuren des Pentaerythrits, des Dipentaerythrits und/oder Tripentaerythrits, wobei weniger als 0.3 Gew.-% C17-Fettsäureester enthalten sind. Die Ester müssen einen Schmelzpunkt von wenigstens 30°C aufweisen. Es kann sich auch um Mischester, beispielsweise langkettiger und kurzkettiger Fettsäuren, handeln, solange sie den geforderten Schmelzpunkt aufweisen. Eine weitere bevorzugte Ausführungsform der O/W-Gelzusammensetzung ist dadurch gekennzeichnet, daß die Wachskomponente (b) ausgewählt ist aus der Gruppe der Ester, die man durch Umsetzung des Pentaerythrits oder Dipentaerythrits mit einem Fettsäuregemisch enthaltend 40 - 50 Gew.-% C16-Fettsäure und 45 - 55 Gew.-% C18-Fettsäure erhält. Besonders bevorzugt sind Ester linearer, unverzweigter C16/C18-Fettsäuren. Eine weitere bevorzugte Ausführungsform der O/W-Gelzusammensetzung enthält wenigstens eine Wachskomponente (b) ausgewählt aus der Gruppe der Ester des Pentaerythrits mit einem Anteil an (a) 5 - 35 Gew.-% Monoester, (b) 20 - 50 Gew.-% Diester und (c) 25 - 50 Gew.-% Triester, und ggf. Tetraester. Ganz besonders bevorzugt sind Ester, die man durch Umsetzung des Pentaerythrits mit einem Fettsäuregemisch enthaltend 40 - 50 Gew.-% C16-Fettsäure und 45 - 55 Gew.-% C18-Fettsäure erhält und die folgende Esterverteilung aufweisen: (a) 12 - 19 Gew.-% Monoester, (b) 25 - 35 Gew.-% Diester und (c) 30 - 40 Gew.-% Triester, und 6 - 11 Gew.-% Tetraester.

### Ölkörper

Die erfindungsgemäßen O/W-Gele enthalten 1 - 30 Gew.-%, bezogen auf die Gesamtzusammensetzung, einer bei 25°C flüssigen Ölkomponente oder eines beliebigen Gemisches dieser Ölkomponenten. Vorzugsweise sind die Ölkomponente(n) in einer Gesamtmenge von 3 - 20 Gew.-%, insbesondere 5 - 15 Gew.-% und besonders bevorzugt 7 - 12 Gew.-% enthalten. Als Ölkörper sind beispielsweise die nachstehend genannten Verbindungsklassen geeignet, soweit diese bei 25°C flüssig sind. Hierzu zählen u.a. Guerbetalkohole auf Basis von Fettalkoholen mit 6 bis 18, vorzugsweise 8 bis 10 Kohlenstoffatomen in Frage, Ester von linearen oder verzweigten, gesättigten oder ungesättigten C₆-C₂₂-Fettsäuren mit linearen oder verzweigten, gesättigten oder ungesättigten C₆-C₂₂-Fettalkoholen, insbesondere 2-Ethylhexanol. Beispielhaft seien genannt Hexyllaurat, Myristylisostearat, Myristyloleat, Cetylisostearat, Cetyloleat, Stearylisostearat, Stearyloleat, Isostearylmyristat, Isostearylpalmitat, Isostearylstearat, Isostearylisostearat, Isostearyloleat, Oleylmyristat, Oleylisostearat, Oleyloleat, Oleylerucat, Erucylisostearat, Erucyloleat, Cococaprylat/caprat. Weitere geeignete Ester sind z.B. Ester von C₁₈-C₃₈-Alkylhydroxycarbonsäuren mit linearen oder verzweigten, gesättigten oder ungesättigten C₆-C₂₂-Fettalkoholen, Ester von linearen und/oder verzweigten, gesättigten oder ungesättigten Fettsäuren mit mehrwertigen Alkoholen (wie z. B. Propylenglycol, Dimerdiol oder Trimertriol) und/oder Guerbetalkoholen, Triglyceride oder Triglyceridmischungen, Mono-/Di-/Triglyceridmischungen, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten, gesättigten oder ungesättigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare Dialkylcarbonate, Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Cetiol® AB), lineare oder verzweigte, symmetrische oder unsymmetrische Dialkylether mit 6 bis 22 Kohlenstoffatomen pro Alkylgruppe, wie z. B. Di-n-octyl-ether (Cetiol® OE) oder Ringöffnungsprodukte von epoxidierten Fettsäureestern mit Polyolen, Kohlenwasserstoffe wie Paraffin- oder Mineralöle, Silikonöle und Oligo- oder Polyalphaolefine.

Dialkylcarbonate und Ester aus C8-C24-Fettsäuren und C8-C24 Fettalkoholen bzw. ein Gemisch dieser Substanzen sind erfindungsgemäß als Ölkörper bevorzugt. Die Dialkylcarbonate können symmetrisch oder unsymmetrisch, verzweigt oder unverzweigt, gesättigt oder ungesättigt sein und lassen sich nach Umesterungsreaktionen, die aus dem Stand der Technik hinlänglich bekannt sind, herstellen.

Erfindungsgemäß besonders geeignet sind Dialkylcarbonate mit Alkylketten, die 6 bis 24 Kohlenstoffatome aufweisen, insbesondere Di-n-octylcarbonat oder Di-(2-ethylhexyl)carbonat oder ein Gemisch dieser Substanzen. Unter diesen ist das Di-n-Octylcarbonat. bevorzugt.

Die erfindungsgemäß einsetzbaren Kohlenwasserstoffe weisen eine Kettenlänge von vorzugsweise 8 bis 40 C-Atomen aus. Sie können verzweigt oder unverzweigt sein, gesättigt oder ungesättigt. Unter diesen sind verzweigte, gesättigte C8-C40-Alkane bevorzugt. Es können sowohl Reinsubstanzen eingesetzt werden als auch Substanzgemische. Üblicherweise handelt es sich um Substanzgemische verschiedener isomerer Verbindungen. Zusammensetzungen, die Alkane mit 10 bis 30, vorzugsweise 12 bis 20, und besonders bevorzugt 16 bis 20 Kohlenstoffatome aufweisen, sind besonders geeignet, und unter diesen ein Gemisch aus Alkanen, welches wenigstens 10 Gew.-% verzweigte Alkane bezogen auf die Gesamtmenge der Alkane enthält. Vorzugsweise handelt es sich um verzweigte, gesättigte Alkane. Besonders gut geeignet sind Gemische aus Alkanen ist, welche mehr als 1 Gew.-% 5,8-Diethyldodecan und/oder mehr als 1 Gew.-% Didecen enthalten.

Eine bevorzugte Ausführungsform der erfindungsgemäßen O/W-Gelzusammensetzung enthält (a) 0,05 - 5 Gew.-% wenigstens eines Polyacrylats, (b) 0,1 -10 Gew.-% wenigstens eines C16/C18-Esters des Pentaerythrits und/oder des Dipentaerythrits, (c) 1 - 30 Gew.-% wenigstens einer Ölkomponente und (d) 60 - 95 Gew.-% Wasser. Das Polyacrylat (a) ist vorzugsweise ein Natriumpolyacrylat. Die Ölkomponenten sind vorzugsweise ausgewählt aus den Fettsäureestern oder Dialkylcarbonaten oder einem beliebigen Gemisch dieser Substanzen.

Eine weitere bevorzugte Ausführungsform ist eine OIW-Gelzusammensetzung mit einem Gehalt an (a) 0,5 - 2 Gew.-% wenigstens eines Natriumpolyacrylats, (b) 0,5 - 2 Gew.-% wenigstens eines Esters, den man durch Umsetzung des Pentaerythrits und/oder des Dipentaerythrits mit einem Fettsäuregemisch enthaltend 40 - 50 Gew.-% C16-Fettsäure und 45 - 55 Gew.-% C18-Fettsäure, erhält, (c) 7 -12 Gew.-% wenigstens einer Ölkomponente ausgewählt aus bei 25 °C flüssigen Fettsäureestern, Trigylceriden, Dialkylcarbonaten, Kohlenwasserstoffen, Dialkylethern oder einem beliebigen Gemisch dieser Substanzen und (d) 60 - 95 Gew.-% Wasser. Vorzugsweise sind die unter (b) genannten Ester Pentaerythritester mit folgender Esterverteilung: 12 - 19 Gew.-% Monoester, 25 - 35 Gew.-% Diester, 30 - 40 Gew.-% Triester und 6 - 11 Gew.-% Tetraester.

Ein weiterer Gegenstand der Anmeldung ist ein Verfahren zur Herstellung der erfindungsgemäßen O/W-Gelzusammensetzung, wobei man entweder a) den Gelbildner oder ein Gemisch der Gelbildner (a) in der flüssigen Ölphase, welche die Wachs- und Ölkomponenten enthält, dispergiert und diese anschliessend mit der wässrigen Phase emulgiert, oder b) eine Quellung des Gelbildners oder eines Gemisches der Gelbildner in der wässrigen Phase vornimmt und diese mit der flüssigen Ölphase vermischt oder c) eine Quellung des Gelbildners oder eines Gemisches der Gelbildner in einem niedrigmolekularen Polyol oder Polyolgemisch mit einem Molekulargewicht < 1000 Dalton mit der wässrigen Phase und mit der flüssigen Ölphase verarbeitet.

Gegenstand der Erfindung ist auch die Verwendung der erfindungsgemäßen O/W-Gelzusammensetzung zur Körperpflege. Ein weiterer Gegenstand der Erfindung ist die Verwendung von Estern des Pentaerythrits, des Dipentaerythrits und/oder Tripentaerythrits zur Verbesserung der Salztoleranz von Gelzusammensetzungen gemäß Anspruch 1.

### Weitere fakultative Hilfs- und Zusatzstoffe

Je nach Applikationszweck enthalten die kosmetischen Formulierungen eine Reihe weiterer Hilfs- und Zusatzstoffe wie beispielsweise Emulgatoren/Tenside, weitere Wachs- oder Lipidkompoenten, weitere Verdickungsmittel, Überfettungsmittel, Stabilisatoren, Polymere, Lecithine, Phospholipide, biogene Wirkstoffe, UV-Lichtschutzfaktoren, Antioxidantien, Deodorantien, Filmbildner, Quellmittel, Insektenrepellentien, Hydrotrope, Solubilisatoren, Konservierungsmittel, Parfümöle, Farbstoffe etc., die nachstehend exemplarisch aufgelistet sind. Die Mengen der jeweiligen Zusätze richten sich nach der beabsichtigten Verwendung.

Als weitere Wachse/Lipidkomponenten können Fette und fettähnlichen Substanzen mit wachsartiger Konsistenz eingesetzt werden. Hierzu gehören u.a. Fette (Triglyceride), Mono- und Diglyceride, natürliche und synthetische Wachse, Fett- und Wachsalkohole, Fettsäuren, Ester von Fettalkoholen und Fettsäuren sowie Fettsäureamide oder beliebige Gemische dieser Substanzen.

Unter Fetten versteht man Triacylglycerine, also die Dreifachester von Fettsäuren mit Glycerin. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Erfindungsgemäß einsetzbar und als Konsistenzgeber besonders gut geeignet sind sogenannte gehärtete Fette und Öle, die durch Partialhydrierung gewonnen werden. Pflanzliche gehärtete Fette und Öle sind bevorzugt, z. B. gehärtetes Rizinusöl, Erdnußöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter und Kokosfett.

Geeignet sind u.a. die Dreifachester von Glycerin mit C₁₂-C₆₀-Fettsäuren und insbesondere C₁₂-C₃₆-Fettsäuren. Hierzu zählt gehärtetes Rizinusöl, ein Dreifachester aus Glycerin und einer Hydroxystearinsäure, der beispielsweise unter der Bezeichnung Cutina^{®} HR im Handel ist. Ebenso geeignet sind Glycerintristearat, Glycerintribehenat (z. B. Syncrowax^{®} HRC), Glycerintripalmitat oder die unter der Bezeichnung Syncrowax^{®} HGLC bekannten Triglycerid-Gemische.

Als zusätzliche Wachskomponenten sind insbesondere Mono- und Diglyceride bzw. Mischungen dieser Partialglyceride einsetzbar. Zu den erfindungsgemäß einsetzbaren Glyceridgemischen zählen die von der Cognis Deutschland GmbH & Co. KG vermarkteten Produkte Novata^{®} AB und Novata^{®} B (Gemisch aus C₁₂-C₁₈-Mono-, Di- und Triglyceriden) sowie Cutina^{®} MD oder Cutina^{®} GMS (Glycerylstearat).

Mischester sowie Mischungen aus Mono-, Di- und Triglyceriden sind erfindungsgemäß bevorzugt geeignet, da sie eine geringere Neigung zur Kristallisation zeigen und somit die Performance der erfindungsgemäßen Zusammensetzung verbessern.

Zu den erfindungsgemäß einsetzbaren Fettalkoholen zählen die C₁₂-C₅₀-Fettalkohole, insbesondere die C₁₂-C₂₄-Fettalkohole, die aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicosanol, Behenylalkohol, Brassidylalkohol, Lignocerylalkohol, Cerylalkohol oder Myricylalkohol. Erfindungsgemäß bevorzugt sind gesättigte unverzweigte Fettalkohole. Erfindungsgemäß einsetzbar sind auch Fettalkoholschnitte, wie sie bei der Reduktion natürlich vorkommender Fette und Öle wie z. B. Rindertalg, Erdnußöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegler-Synthese (Alfole^{®}) oder die teilweise verzweigten Alkohole aus der Oxosynthese (Dobanole^{®}) verwendet werden. Erfindungsgemäß bevorzugt geeignet sind C₁₄-C₁₈-Fettalkohole, die beispielsweise von der Cognis Deutschland GmbH unter der Bezeichnung Lanette^{®} 16 (C₁₆-Alkohol), Lanette^{®} 14 (C₁₄-Alkohol), Lanette^{®} O (C₁₆/C₁₈-Alkohol) und Lanette^{®} 22 (C₁₈/C₂₂-Alkohol) vermarktet werden. Fettalkohole verleihen den Zusammensetzungen ein trockeneres Hautgefühl als Triglyceride und sind daher gegenüber letzteren bevorzugt.

Als zusätzliche Wachskomponenten können auch C₁₄-C₄₀-Fettsäuren oder deren Gemische eingesetzt werden. Hierzu gehören beispielsweise Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-Hydroxystearinsäure, und die Amide oder Monoethanolamide der Fettsäuren, wobei diese Aufzählung beispielhaften und keinen beschränkenden Charakter hat.

Erfindungsgemäß verwendbar sind beispielsweise **natürliche pflanzliche Wachse,** wie Candelillawachs, Carnaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und tierische Wachse, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die **Mineralwachse,** wie z. B. Ceresin und Ozokerit oder die petrochemischen Wachse, wie z. B. Petrolatum, Paraffinwachse und Mikrowachse. Als Wachskomponente sind auch **chemisch modifizierte Wachse,** insbesondere die Hartwachse, wie z. B. Montanesterwachse, Sasolwachse und hydrierte Jojobawachse einsetzbar. Zu den **synthetischen Wachsen,** die erfindungsgemäß einsetzbar sind, zählen beispielsweise wachsartige Polyalkylenwachse und Polyethylenglycolwachse. Pflanzliche Wachse sind erfindungsgemäß bevorzugt.

Die zusätzliche Wachskomponente kann ebenso gewählt werden aus der Gruppe der Wachsester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, aus der Gruppe der mit Carbonsäuren veresterten Polyole (andere als Pentaerythrit, Dipentaerythrit oder Tripentaerythrit), sowie ferner aus der Gruppe der Lactide langkettiger Hydroxycarbonsäuren. Beispiel solcher Ester sind die C₁₆-C₄₀-Alkylstearate, C₂₀-C₄₀-Alkylstearate (z. B. Kesterwachs^{®} K82H), C₂₀-C₄₀-Dialkylester von Dimersäuren, C₁₈-C₃₈-Alkylhydroxystearoylstearate oder C₂₀-C₄₀-Alkylerucate. Ferner sind C₃₀-C₅₀-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglykoldi(12-hydroxystearat), Stearylstearat, Palmitylstearat, Stearylbehenat, Cetearylbehenat und Behenylbehenat einsetzbar.

Als **oberflächenaktive Stoffe** können anionische, nichtionische, kationische und/oder amphotere bzw. zwitterionische Tenside bzw. Emulgatoren oder ein beliebiges Gemisch dieser Tenside/Emulgatoren enthalten sein. Der Gehalt oberflächenaktiver Substanzen hängt von der Art der Formulierung ab, übersteigt aber üblicherweise 10 Gew.-% nicht. Eine bevorzugte Ausführungsform der Erfindung enthält keine anionischen oder kationischen Tenside, weist aber einen Gehalt an nichtionischen Tensiden auf. Die nichtionischen Tenside können u.a. auch in den handelsüblichen Gelbildnem enthalten sein. Der bevorzugte Gehalt an nichtionischen Tensiden liegt zwischen 0 - 5 Gew.-%, insbesondere 0,1 - 3 Gew.-% bezogen auf die Gesamtzusammensetzung des Gels.

Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Polyglycerinester, Alkylphenolpolyglycolether, Fettsäurepolyglycolester, Fettsäureamid-polyglycolether, Fettaminpolyglycolether, alkoxylierte Triglyceride, Mischether bzw. Mischformale, Alk(en)yloligoglykoside bzw. Glucoronsäurederivate - gegebenenfalls partiell oxidiert, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d. h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

Als weitere **Verdickungsmittel** eignen sich beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, Polyvinylalkohol, Polyvinylpyrrolidon und Bentonite wie z. B. Bentone^{®} Gel VS-5PC (Rheox).

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z. B. Wärme wieder abzugeben. UV-B-Filter können öllöslich oder wasserlöslich sein. Als typische UV-A-Filter kommen insbesondere Derivate des Benzoylmethans in Frage. Die UV-A und UV-B-Filter können selbstverständlich auch in Mischungen eingesetzt werden, z.B. Kombinationen aus den Derivaten des Benzoylmethans, z. B. 4-tert.-Butyl-4'-methoxydibenzoylmethan (Parsol^{®} 1789) und 2-Cyano-3,3-phenylzimtsäure-2-ethyl-hexylester (Octocrylene) sowie Estern der Zimtsäure, vorzugsweise 4-Methoxyzimtsäure-2-ethylhexylester und/oder 4-Methoxyzimtäurepropylester und/oder 4-Methoxyzimtsäureisoamylester. Häufig werden derartige Kombinationen mit wasserlöslichen Filtern wie z. B. 2-Phenylbenzimidazol-5-sulfonsäure und deren Alkali-, Erdalkali-, Ammonium-, Alkylammonium-, Alkanolammonium- und Glucammoniumsalze kombiniert.
Neben den genannten löslichen Stoffen kommen auch unlösliche Lichtschutzpigmente, nämlich feindisperse Metalloxide in Frage. Beispiele für geeignete Metalloxide sind insbesondere Zinkoxid und Titandioxid.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt.

Unter **biogenen Wirkstoffen** sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

**Desodorierende Wirkstoffe** wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend eignen sich als deosodorierende Wirkstoffe u.a. keimhemmende Mittel, Enzyminhibitoren, Geruchsabsorber oder Geruchsüberdecker.

Als **Insekten-Repellentien** kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent^{®} 3535 von der Merck KGaA vertrieben wird, sowie Butylacetylaminopropionate in Frage.

Als Selbstbräuner eignet sich Dihydroxyaceton. Als Tyrosininhibitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Zur Verbesserung des Fließvarhaltens können ferner **Hydrotrope,** wie beispielsweise Ethanol, Iso-propylalkohol, oder Polyole eingesetzt werden. Polyole, die hier in Betracht kommen, besitzen vorzugsweise 2 bis 15 Kohlenstoffatome und mindestens zwei Hydroxylgruppen.

Als **Konservierungsmittel** eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Als **Parfümöle** seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Als **Farbstoffe** können die für kosmetische Zwecke geeigneten und zugelassenen Substanzen verwendet werden. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Diese Farbstoffe werden üblicherweise in Konzentrationen von 0,001 bis 0,1 Gel.-%, bezogen auf die gesamte Mischung, eingesetzt.

### Beispiele

Die angegebenen Mengen beziehen sich auf Gew.-% der handelsüblichen Substanz in der Gesamtzusammensetzung.

| **Inhalfsstoff Handelsname / INCI** | **V1** | **V2** | **V3** | **1** | **2** | **3** | **4** | **5** |
|---|---|---|---|---|---|---|---|---|
| | | | | | | | | |
| **Cosmedia® SP** Natriumpolyacrylat | 1.0 | | 1,0 | 1,0 | 1.0 | 1.0 | 1,0 | |
| **Sepigel**® **305** Polyacrylamid (und) C13-14 Isoparaffin (und) Laureth-7 | | 0,3 | | | | | | 0,3 |
| Pentaerythrityl Distearat¹⁾ | | | | 1,0 | 1.0 | 2.0 | 1,0 | 1,0 |

| **Dow Corning® 245** Cyclomethicon | | | | | | | | |
|---|---|---|---|---|---|---|---|---|
| Ethanol | | | | | | | 15,0 | |
| Natriumchlorid | | | 0,5 | 0,5 | | | | |
| **Cetiol® CC** Dicaprylyl Carbonat | 5.0 | 5,0 | 5,0 | 5,0 | 5.0 | 5.0 | 5,0 | 5,0 |
| **Cetiol® LC**Coco-Caprylat / Caprat | 5.0 | 5,0 | 5,0 | 5,0 | 5.0 | 5.0 | 5,0 | 5,0 |
| Wasser | ad 100 | ad 100 | ad 100 | ad 100 | ad 100 | ad 100 | ad 100 | ad 100 |
| | | | | | | | | |
| **Viskosität bei 20°C nach 1 Woche** | 125000 | 37500 | - | 5200 | 162500 | 187500 | 125000 | 50000 |

| **Phasenstabilität bei -5°C/ 20°C /40°C** | | | | | | | | |
|---|---|---|---|---|---|---|---|---|
| nach 1 Woche | 1/1/1 | 1/1/1 | 5/5/5 | 1/1/1 | 1/1/1 | 1/1/1 | 1/1/1 | 1/1/1 |
| nach 4 Wochen | 1/1/1 | | 5/5/5 | | 1/1/1 | 1/1/1 | 1/1/1 | |
| nach 12 Wochen | 1/1/1 | | 5/5/5 | | 1/1/1 | 1/1/1 | | |
| | | | | | | | | |

| **Sensorische Bewertung** | | | | | | | | |
|---|---|---|---|---|---|---|---|---|
| Glätte | - | - | - | | ++ | + | ++ | ++ |
| Weichheit | - | - | - | | ++ | + | ++ | + |
| Akzeptanz | - | - | - | | ++ | + | ++ | + |

| | | | | | | | | |
|---|---|---|---|---|---|---|---|---|
| 1) Das Pentaerythrityl Distearat ist ein Estergemisch, das durch Umsetzung von Pentaerythrit mit einem Fettsäuregemisch enthaltend 42 - 48 Gew.-% C16-Fettsäure und 50 - 56 Gew.-% C18-Fettsäure (restliche Mengen: ≤ C14-Fettsäuren und > C18-Fettsäuren) erhalten wird und folgende Esterverteilung aufweist: 12 -19 Gew.-% Monoester, (b) 25 - 35 Gew.-% Diester, (c) 30 - 40 Gew.-% Triester und (d) 6 -11 Gew.-% Tetraester und weniger als 0,3 Gew.-% C17-Fettsäureacylgruppen enthält. **Viskositätsmessung** - Bei V1, V2, und den Beispielen 2 - 6: 20°C, Brookfield-Viskosimeter RVF, Spindel TE mit Helipath bei 4 Umdrehungen pro Minute. - Bei V3 und Beispiel 1: 23°C, Brookfield-Viskosimeter RVF, Spindel 5 bei 10 Umdrehungen pro Minute **Visuelle Beurteilung der Phasenstabilität** 1 = stabil; 2 = sehr geringfügige Trennung; 3 = geringe Trennung; 4 = deutliche Trennung; 5 = vollständig getrennt **Sensorische Bewertung:** ++= exzellent; + = sehr gut; 0 = gut, - = mittel; --= unbefriedigend Testgruppe:10 erfahrene und geschulte Freiwillige | | | | | | | | |

10µl der o.g. Zusammensetzungen wurden mittels einer Mikropipette auf die unbehaarte Seite der Unterarme der Probanden appliziert und mit den Fingern der Hände der kontralateralen Seite eingerieben. Die Beurteilung der Sensorik erfolgte während und nach der Absorption.

Der Sensorik-Test wurde an 10 Probanden durchgeführt, wie in dem Buch "Cosmetic Lipids and the Skin Barrier" (Marcel Dekker Verlag New York, 2002, Ed. Thomas Förster, S. 319-352) beschrieben.

## Patentansprüche

1. O/W-Gelzusammensetzung enthaltend
(a) 0,05 - 5 Gew.-% wenigstens eines polymeren Gelbildners ausgewählt aus der Gruppe der Homopolymere oder Copolymere von Acrylsäure und/oder Acrylamid und deren Derivaten,
(b) 0,1 -10 Gew.-% wenigstens einer Wachskomponente mit einem Schmelzpunkt von wenigstens 30°C ausgewählt aus der Gruppe der Pentaerythritester, der Dipentaerythritester und/oder der Tripentaerythritester
(c) 1- 30 Gew.-% wenigstens einer bei 25°C flüssigen Ölkomponente und
(d) 60 - 95 Gew.-% Wasser.

2. O/W-Gelzusammensetzung gemäß Anspruch 1, **dadurch gekennzeichnet, dass** sie keine anionischen und kationischen Emulgatoren/Tenside enthält.

3. O/W-Gelzusammensetzung gemäß Anspruch 1 oder 2, **dadurch gekennzeichnet, dass** die Wachskomponente (b) in einer Menge von 0,2 - 5 Gew.-% der Gesamtzusammensetzung enthalten ist.

4. O/W-Gelzusammensetzung gemäß wenigstens einem der Ansprüche 1 bis 3, **dadurch gekennzeichnet, dass** die Wachskomponente (b) ausgewählt ist aus der Gruppe der Ester gesättigter oder ungesättigter und/oder verzweigter oder unverzweigter C6-C24-Fettsäuren, vorzugsweise C14-C22-Fettsäuren des Pentaerythrits, des Dipentaerythrits und/oder des Tripentaerythrits oder einem beliebigen Gemisch dieser Ester, wobei weniger als 0,3 Gew.-% C17-Fettsäureester enthalten sind.

5. O/W-Gelzusammensetzung gemäß Anspruch 4, **dadurch gekennzeichnet, daß** die Wachskomponente (b) ausgewählt ist aus der Gruppe der Ester, die man durch Umsetzung des Pentaerythrits oder des Dipentaerythrits mit einem Fettsäuregemisch enthaltend 40 - 50 Gew.-% C16-Fettsäure und 45 - 55 Gew.-% C18-Fettsäure, erhält.

6. O/W-Gelzusammensetzung gemäß Anspruch 5, **dadurch gekennzeichnet, daß** die Wachskomponente (b) ausgewählt ist aus der Gruppe der Ester des Pentaerythrits mit einem Anteil an (a) 5 - 35 Gel.-% Monoester, (b) 20 - 50 Gew.-% Diester und (c) 25 - 50 Gew.-% Triester, und ggf. Tetraester.

7. O/W-Gelzusammensetzung gemäß wenigstens einem der Ansprüche 1 bis 6, **dadurch gekennzeichnet, dass** sie
(a) 0,05 - 5 Gew.-% wenigstens eines Polyacrylats,
(b) 0,1-10 Gew.-% wenigstens eines C16/C18-Esters des Pentaerythrits und/oder des Dipentaerythrits,
(c) 1 - 30 Gew.-% wenigstens einer bei 25°C flüssigen Ölkomponente und
(d) 60 - 95 Gew.-% Wasser
enthält.

8. O/W-Gelzusammensetzung gemäß Anspruch 7, **dadurch gekennzeichnet, dass** das Polyacrylat (a) ein Natriumpolyacrylat ist.

9. O/W-Gelzusammensetzung gemäß wenigstens einem der Ansprüche 7 bis 8, **dadurch gekennzeichnet, dass** die Ölkomponenten ausgewählt sind aus den Fettsäureestern oder Dialkylcarbonaten oder einem beliebigen Gemisch dieser Substanzen.

10. O/W-Gelzusammensetzung gemäß wenigstens einem der Ansprüche 1 bis 9, **dadurch gekennzeichnet, dass** sie bei 20°C eine Viskosität von 50000 bis 500000 mPa·s aufweist, gemessen mit einem Brookfield-Viskosimeter RVF, Spindel TE mit Helipath bei 4 Umdrehungen pro Minute.

11. Verfahren zur Herstellung O/W-Gelzusammensetzung gemäß wenigstens einem der Ansprüche 1 bis 10, **dadurch gekennzeichnet, dass** man dass man entweder a) den Gelbildner oder ein Gemisch der Gelbildner in der flüssigen Ölphase, welche die Wachs- und Ölkomponenten enthält, dispergiert und diese anschliessend mit der wässrigen Phase emulgiert, oder b) eine Quellung des Gelbildners oder eines Gemisches der Gelbildner in der wässrigen Phase vornimmt und diese mit der flüssigen Ölphase vermischt oder c) eine Quellung des Gelbildners oder eines Gemisches der Gelbildner in einem niedrigmolekularen Polyol oder Polyolgemisch mit einem Molekulargewicht < 1000 Dalton mit der wässrigen Phase und mit der flüssigen Ölphase verarbeitet.

12. Verwendung einer O/W-Gelzusammensetzung gemäß wenigstens einem der Ansprüche 1 bis 10 zur kosmetischen Körperpflege.

13. Verwendung von Estern des Pentaerythrits, des Dipentaerythrits und/oder Tripentaerythrits zur Verbesserung der Salztoleranz von Gelzusammensetzungen mit Gelbildnem gemäß Anspruch 1.

## Claims

1. O/W gel composition containing
(a) 0.05 to 5% by weight of at least one polymeric gel former selected from the group of homopolymers or copolymers of acrylic acid and/or acrylamide and derivatives thereof,
(b) 0.1 to 10% by weight of at least one wax component with a melting point of at least 30°C selected from the group of pentaerythritol esters, dipentaerythritol esters and/or tripentaerythritol esters,
(c) 1 to 30% by weight of at least one oil component liquid at 25°C and
(d) 60 to 95% by weight of water.

2. O/W gel composition as claimed in claim 1, **characterized in that** it does not contain any anionic or cationic emulsifiers/surfactants.

3. O/W gel composition as claimed in claim 1 or 2, **characterized in that** the wax component (b) is present in a quantity of 0.2 to 5% by weight, based on the composition as a whole.

4. O/W gel composition as claimed in at least one of claims 1 to 3, **characterized in that** the wax component (b) is selected from the group of esters of saturated or unsaturated and/or branched or unbranched C₆₋₂₄ fatty acids - preferably C₁₄₋₂₂ fatty acids - of pentaerythritol, dipentaerythritol and/or tripentaerythritol or mixtures thereof, less than 0.3% by weight C₁₇ fatty acid esters being present.

5. O/W gel composition as claimed in claim 4, **characterized in that** the wax component (b) is selected from the group of esters which are obtained by reaction of pentaerythritol or dipentaerythritol with a fatty acid mixture containing 40 to 50% by weight C₁₆ fatty acid and 45 to 55% by weight C₁₈ fatty acid.

6. O/W gel composition as claimed in claim 5, **characterized in that** the wax component (b) is selected from the group of esters of pentaerythritol with a percentage content of (a) 5 to 35% by weight monoesters, (b) 20 to 50% by weight diesters and (c) 25 to 50% by weight triesters and optionally tetraesters.

7. O/W gel composition as claimed in at least one of claims 1 to 6, **characterized in that** they contain
(a) 0.05 to 5% by weight of at least one polyacrylate,
(b) 0.1 to 10% by weight of at least one C_{16/18} ester of pentaerythritol and/or dipentaerythritol,
(c) 1 to 30% by weight of at least one oil component and
(d) 60 to 95% by weight water.

8. O/W composition as claimed in claim 7, **characterized in that** the polyacrylate (a) is a sodium polyacrylate.

9. O/W gel composition as claimed in at least one of claims 7 to 8, **characterized in that** the oil components are selected from the fatty acid esters or dialkyl carbonates or mixtures thereof.

10. O/W gel composition as claimed in at least one of claims 1 to 9, **characterized in that** it has a viscosity at 20°C of 50,000 to 500,000 mPa.s, as measured with a Brookfield RVF viscosimeter, spindle TE with Helipath, at 4 r.p.m.

11. Process for the production of the o/w gel composition claimed in at least one of claims 1 to 10, **characterized in that** either a) the gel former or a mixture of the gel formers (a) is dispersed in the liquid oil phase containing the wax and oil components and the resulting dispersion is subsequently emulsified with the aqueous phase or b) the gel former or a mixture of the gel formers is swollen in the aqueous phase and the whole is mixed with the liquid oil phase or c) the gel former or a mixture of the gel formers is swollen in a low molecular weight polyol or polyol mixture with a molecular weight of < 1,000 dalton and the whole is processed with the aqueous phase and with the liquid oil phase.

12. Use of the O/W gel composition claimed in at least one of claims 1 to 10 for cosmetic body care.

13. Use of esters of pentaerythritol, dipentaerythritol and/or tripentaerythritol for improving the salt tolerance of gel compositions containing the gel formers claimed in claim 1.

## Revendications

1. Composition en gel H/E contenant
(a) 0,05-5 % en poids d'au moins un agent gélifiant polymère choisi dans le groupe des homopolymères ou copolymères d'acide acrylique et/ou d'acrylamide et de leurs dérivés,
(b) 0,1-10 % en poids d'au moins un composant de type cire ayant un point de fusion d'au moins 30 °C, choisi dans le groupe des esters de pentaérythritol, des esters de dipentaérythritol et/ou des esters de tripentaérythritol
(c) 1-30 % en poids d'au moins un composant huileux liquide à 25 °C et
(d) 60-95 % en poids d'eau.

2. Composition en gel H/E selon la revendication 1, **caractérisée en ce qu'**elle ne contient pas d'émulsifiants/tensioactifs cationiques.

3. Composition en gel H/E selon la revendication 1 ou 2, **caractérisée en ce que** le composant de type cire (b) est contenu en une quantité de 0,2 - 5 % en poids de la composition.

4. Composition en gel H/E selon au moins l'une des revendications 1 à 3, **caractérisée en ce que** le composant de type cire (b) est choisi dans le groupe constitué par les esters d'acides gras en C₆-C₂₄, de préférence d'acides gras en C₁₄-C₂₂, saturés ou insaturés et/ou ramifiés ou non ramifiés, du pentaérythritol, du dipentaérythritol et/ou du tripentaérythritol ou un mélange quelconque de ces esters, contenant moins de 0,3 % en poids d'esters d'acide gras en C₁₇.

5. Composition en gel H/E selon la revendication 4, **caractérisée en ce que** le composant de type cire (b) est choisi dans le groupe des esters qu'on obtient en faisant réagir le pentaérythritol ou le dipentaérythritol avec un mélange d'acides gras contenant 40-50 % en poids d'acide gras en C₁₆ et 45-55 % en poids d'acide gras en C₁₈.

6. Composition en gel H/E selon la revendication 5, **caractérisée en ce que** le composant de type cire (b) est choisi dans le groupe des esters du pentaérythritol ayant une teneur en (a) de 5 - 35 % en poids de monoesters, (b) 20 - 50 % en poids de diesters et (c) 25 - 50 % en poids de triesters et éventuellement tétraesters.

7. Composition en gel H/E selon au moins l'une des revendications 1 à 6, **caractérisée en ce qu'**elle contient
(a) 0,05-5 % en poids d'au moins un polyacrylate,
(b) 0,1-10 % en poids d'au moins un ester de C₁₆/C₁₈ du pentaérythritol et/ou du dipentaérythritol,
(c) 1-30 % en poids d'au moins un composant huileux liquide à 25 °C et
(d) 60-95 % en poids d'eau.

8. Composition en gel H/E selon la revendication 7, **caractérisée en ce que** le polyacrylate (a) est un polyacrylate de sodium.

9. Composition en gel H/E selon au moins l'une des revendications 7 et 8, **caractérisée en ce que** le composant huileux est choisi parmi les esters d'acides gras ou les carbonates de dialkyle ou est un mélange quelconque de ces substances.

10. Composition en gel H/E selon au moins l'une des revendications 1 à 9, **caractérisée en ce qu'**elle présente à 20 °C une viscosité de 50 000 à 500 000 mPa.s, mesurée à l'aide d'un viscosimètre Brookfield RVF, broche TE à entraînement Helipath à 4 tours/minute.

11. Procédé pour la préparation d'une composition en gel H/E selon au moins l'une des revendications 1 à 10, **caractérisé en ce que** soit a) on disperse l'agent gélifiant ou un mélange des agents gélifiants dans la phase huileuse liquide qui contient le composant huileux et le composant de type cire, et le disperse ensuite avec la phase aqueuse, soit b) on effectue un gonflement de l'agent gélifiant ou d'un mélange des agents gélifiants dans la phase aqueuse et on mélange celle-ci avec la phase huileuse liquide ou c) on effectue un gonflement de l'agent gélifiant ou d'un mélange des agents gélifiants dans un polyol ou mélange de polyols de faible masse moléculaire ayant une masse moléculaire < 1 000 daltons, avec la phase aqueuse et avec la phase liquide.

12. Utilisation d'une composition en gel H/E selon au moins l'une des revendications 1 à 10, pour le soin cosmétique du corps.

13. Utilisation d'esters du pentaérythritol, du dipentaérythritol et/ou du tripentaérythritol pour l'amélioration de la tolérance au sel de compositions en gel à l'aide d'agents gélifiants selon la revendication 1.
